# EUROPEAN PATENT SPECIFICATION

(11) **EP 1 923 091 B1**
(45) Date of publication and mention of the grant of the patent: **02.11.2011**
(21) Application number: 07022026.4
(22) Date of filing: 13.11.2007
(51) Int. Cl.: A61M 16/06

(54) **Nebulizer mask for delivery of aerosolized and nebulized medications**
Verneblermaske zur Verabreichung von Aerosol- und vernebelten Medikamenten
Masque de nébuliseur pour la distribution de médicaments en aérosol et sous forme nébulisée

(30) Priority: 14.11.2006 US 865685 P
(43) Date of publication of application: 21.05.2008
(73) Proprietor: Southmedic Incorporated, Barrie, Ontario L4M 5K3 (CA)
(72) Inventor: Hajgato, Julius, RR2 Shanty Bay Ontario L0L 2L0 (CA); McDonald, Lee, Barrie Ontario L4M 7S5 (CA)
(74) Representative: Beyer, Andreas

(56) References cited:
- WO-A-99/30760
- US-A- 5 813 423
- US-A1- 2002 195 107
- US-A1- 2003 127 102
- US-A1- 2003 145 859
- US-A1- 2006 196 510

## Description

The invention relates medical devices and equipment. Specifically, it relates to a face mask for delivering drugs in aerosol or nebulized form, to the nose and mouth of a patient, for administration to the patient by inhalation.

Face masks that cover the nose and mouth of a patient are used in a variety of medical applications, including delivery of medications in aerosol or vapour form for delivery to a patient's lungs. For example, anesthesia may be delivered in this manner, as well as drugs delivered over a longer time frame to a conscious and alert patient, such as Ventalin™ and other drugs for treating asthma or COPD. Doses of such drugs may be delivered by a nebulizer, in particular when the patient is in a medical setting. Administration of certain drugs in this form is particularly appropriate for children, who may have difficulty in self-administration of such drugs.

Delivery of drugs in nebulized form is carried out primarily with the use of a face mask configured to cover the nose and mouth of the patient, and typically held in place by straps or other means to retain the mask against the patient's face. The typical mask includes an inlet for delivery of the nebulized drug under pressure, the inlet being attached to a drug dispensing device for generating a pressurized gas stream laden with a measured dose of the drug in aerosolized or nebulized form. The nebulizer pumps a flow of air or oxygen through a liquid medicine to turn it into a vapor, which is then inhaled by the patient. An atomizer converts the liquid into a spray or mist and in some cases a fine powder.

Examples of prior art of face masks for delivering nebulized or atomized drugs are described in Canadian Patent Application Nos. 2,542,722 (Smalldone) and 2,447,591 (Smalldone); and U.S. 4,938,209 (Fry). In general, prior art systems include a mask body for covering the nose and mouth of the patient, and a generally central inlet to receive a flow of drug-containing gas, for delivery under pressure to the mask interior. Openings in the mask are provided to discharge gases not inhaled by the patient, and as well to permit discharge of exhaled breath from the patient. Openings are typically provided at an upper region of the mask on opposing sides thereof, and in some cases also one or more openings near the base of the mask.

US 5,813,423 discloses an inhaltor mask adaptable for use with an adult or child. The mask has an indication printed on a surface of the mask for directing emergency personnel to orient the mask in different directions when using the mask for a child and an adult. The mask has a wide end, corresponding to a portion to be placed over an adult's mouth, and a narrow end, corresponding to a portion to be placed over an adult's nose. The mask includes a first hole near the centre of the mask for introducing inhalation air under pressure. The mask further includes a second hole located below the first hole for introducing oxygen into the mask.

US 2006/0196510 discloses a mask for delivery of oxygen to a patient. The mask may have a generally concave wall and base, and has an open structure to permit collapsing of the mask and to permit the patient to function normally while wearing the mask. An oxygen delivery aperture is located in a central portion of the mask, and in the aperture is located a diffuser and a baffle configured to disrupt the air flow from the aperture such that the airflow delivered to the mask enters the mask with a turbulent flow, and wherein an oxygen plume surrounds the patient's nose and mouth. The mask may have an opening in the lower section of the mask. Large, openings in the mask allow exhaled air to be easily discharged, enhancing the patients comfort level and contributing to avoiding occurrences of claustrophobia.

US 2003/00145859 discloses a nasal or full-face mask for pediatric ventilation therapy, having a shallow concave shell and an inlet located near the centre of the mask. Exhalation ports are located on a surface of a tube for introducing air into the mask, and around the periphery of the mask.

WO99/30760 discloses an apparatus for ensuring the fit of a face mask to the face of a patient, by sensing the flow rate of gas drawn through an inlet in the mask while the patient inhales.

It is desirable to provide a face mask in which gas leakage to the environment outside of the mask is minimized, and in which leaked gas is directed away from the patient's eyes and in a manner that minimizes contamination of the environment surrounding the patient and others within the immediate environment of the patient. Reduced leakage of gas also promotes more efficient drug delivery, with Increased concentration of nebulized drug being delivered to the patient, and as well less variability and more precise drug delivery.

These problems are solved by a mask as defined in claim 1.

A further aspect of masks intended for pediatric use relates to the acceptance by and willingness of young patients to wear such a mask, in particular for an extended period. There is thus a need to not only improve their comfort, but also to add features that render them less intimidating and "medical" in outward appearances.

It has been found that structural factors of a gas delivery mask influence flow patterns of gas discharged into the interior of a mask. In essence, a primary gas flow is generated by gas entering the mask through the nozzle, and secondary gas flows result from the primary flow contacting the patient's face and rebounding within the mask interior. As well, additional gas flow patterns are generated by the patient's exhalation. Controlling the flow patterns within the mask interior can assist in channeling such gas flows in a desired direction as they exit the mask, including a direction that minimizes the amount of released gas coming into contact with the patient's eyes. The present inventors have found that discharged gas that is directed in a downwards direction from the mask tends not to diffuse through the immediate environment, and results in a reduction in the content of drug-containing gas entering the ambient air at a location likely to be inhaled by adults in the patient's immediate environment. For example, the ambient air around the patient's head tends to be reasonably free of excess drug-containing gases, providing a benefit for caregivers who may bring their own face close to that of the patient. Drug-laden exhaust gases will thus tend to dwell at a lower position within the patient's environment, away from the faces of others in the room.

In one aspect, the invention relates to a face mask for delivery of an aerosolized or nebulized drug to a patient, in the form of a drug-laden gas delivered under pressure. The mask consists of a mask body configured for covering the nose and mouth of the patient and conforming to the shape of a human face. The mask body is configured to provide an interior space when worn by a patient between the wall of the mask and the patient's face. The mask includes a generally centrally disposed inlet, centered within the mask body over the patient's nose, for discharging gas in a substantially horizontal direction towards the patient's face when the mask is upright, such as when worn by a patient in an upright position. The inlet includes a fitting which projects horizontally outwardly for connection with a gas conduit such as a flexible gas tube. The mask body consists of an upper portion extending vertically upwardly from the inlet, and a lower portion extending below the inlet. The upper portion of the mask body is solid, in that it does not include any openings capable of releasing gas into the ambient air. Preferably, only a single opening is provided to release gas from the mask interior, located in the lower portion opposed to the patient's mouth.

The fitting may connect with or consist of an elbow-shaped tube, the horizontal portion of which enters the mask at the inlet, with the vertical portion for attachment to a gas delivery hose leading from a drug nebulizer or atomizer. The gas entering the mask is thus discharged in a horizontal direction (when the mask is upright) for contacting a patient's face in a non-oblique direction. The mask body is preferably configured to generate a helical flow of gas within interior of the mask body after the discharged gas contacts the patient's face. It is believed that the horizontal orientation of the incoming gas tube assists in the generation of a suitable helical gas flow pattern that tends to reduce gas pressure at the margins of the mask. The helical flow pattern is also generated by configuring the mask.

The elbow permits attachment of the gas supply tube in a convenient downward direction from the mask.

The mask body includes a rim for contacting the patient's face, which preferably is pliable so as to form a seal between the patient's face and the mask body. The rim fully surrounds at least the upper portion of the mask body, and preferably surrounds the entire mask body, such that contact with the patient's face is made around all or substantially all of the mask body. The seal formed by the rim prevents or minimize gas leakage from the mask interior upwardly towards the patient's eyes. The mask body includes an upper portion having a substantially triangular shape when seen in plan (front or rear) view such that the opposing rim portions on either side of the mask converge towards the upper end of the mask. The apex thereof is rounded so as to conveniently fit over the bridge of the patient's nose. Further, the rim cants forwardly away from the patient's face at its upper portion, so as to accommodate the projecting nose bridge region of the patient's face.

The interior of the mask body tapers inwardly towards the central inlet, with the wall of the mask being convexly curved when seen from the outside of the mask. It is hypothesized that this curved configuration promotes a helical flow pattern that results in a gas pressure being generated at the margins of the mask which is at atmospheric pressure or close thereto. However, the inventors do not intend to be restricted to this theory. As a result of the gas flow patterns generated within the mask, gas leakage around the mask periphery is minimized, in particular at the upper portion of the mask body adjacent to the patient's eyes. In practice, it is believed that when the mask of the present invention is used, gases discharged from the nozzle may be either directly inhaled by the patient or will contact a portion of the patient's face and rebound towards the inside surface of the mask body. Due to the curved shape of the mask body, when gas particles strike the inside surface of the mask body, it is believed that they tend to be deflected in a manner such that their bulk behavior follows a generally circular or spiraling (helical) pattern, which directs the gas flow away from the mask margins at the rim portion of the mask. Thus, even if a gap exists between the rim and the patient's face, gas flow within the mask interior will tend to be directed away from such gap.

The mask body also includes an array of ribs protruding inwardly towards the user's face, into the interior space defined by the mask body. The ribs radiate outwardly from the central inlet. Preferably, three generally equally spaced ribs are provided. Preferably, a generally triangular wall surrounds the inlet, with the ribs joining with and radiating outwardly from the wall. The ribs serve to stiffen the mask body, and it is also believed that they assist in generating a helical flow pattern for the gas plume within the inside space. In particular, it is believed that the ribs effectively disrupt the gas flow into and direct the plume into separate regions defined by the spaces between the ribs. If three equally spaced ribs are provided, the gas flow will be effectively channeled into three separate regions.

The mask body includes an opening, which preferably is relatively large, at or near the lower portion of the mask, below the nozzle and generally facing the patient's mouth. This opening permits discharge of gas from within the interior of the mask body.

These and other aspects of the present invention are more fully described in the detailed description presented herewith. It will be noted that although one or more specific embodiments as described in detail herein, such embodiments and the features described therein are not intended to limit the scope or spirit of the present invention, but rather only to serve as a particular illustration of the invention.

Directional references presented herein, whether in the specification or claims, for convenience refer to the mask when positioned in its upright position, that is, when worn by a patient in an upright position. Hence, the directional terms such as "upper," "lower," and so forth refer to the mask in the normal upright position as if worn by a patient in the standing or upright sitting position. "Forward" refers to the direction facing away from the patient's face, while "rearward" refers to the direction towards the patient's face. All expressions such as "horizontal", "vertical" and the like are understood to contemplate reasonable departures therefrom. To the extent any dimensions are presented by way of numerical values, it will be understood that variations in the order of 10% from such dimensions are contemplated. However, dimensions are presented herein merely by way of example and are not intended to limit the scope of the invention.
- FIGURE 1: is a perspective view of a nebulizer mask according to the present invention, on a mannequin head;

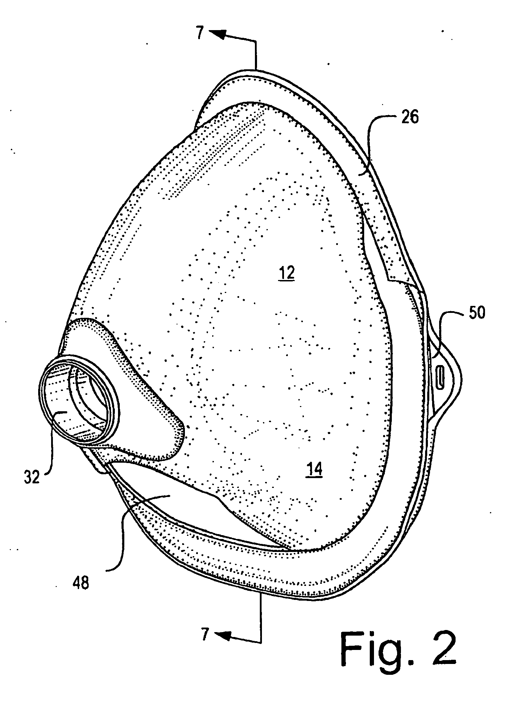

- FIGURE 2: is a side perspective view of the mask showing the exterior of the mask, with the elbow-shaped fitting removed;
- FIGURE 3: is a rear perspective view thereof showing the interior of the mask;
- FIGURE 4: is a perspective view, generally from the side, showing the exterior of the mask and a nozzle attached thereto;
- FIGURE 5: is a perspective view thereof, generally from the side, showing the exterior of the mask and a nozzle and headgear attached thereto;
- FIGURE 6: is a front elevational view of the mask, showing an alternative embodiment intended for pediatric use;
- FIGURE 7: is a cross-sectional view of the mask along line 7-7 in Figure 2.

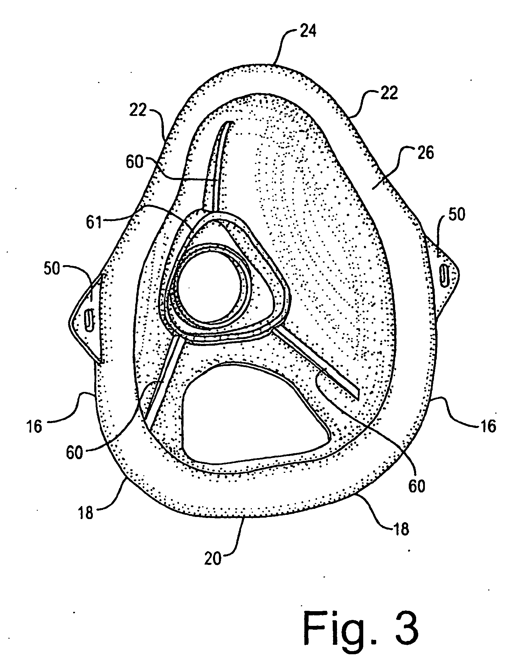

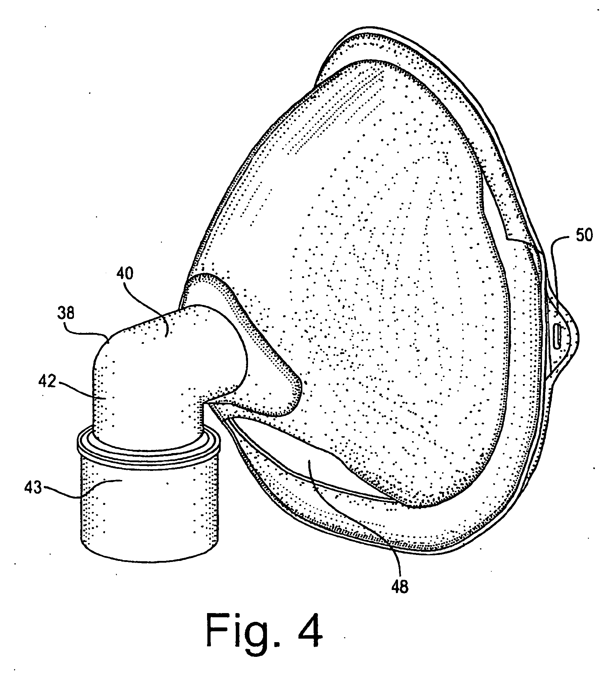

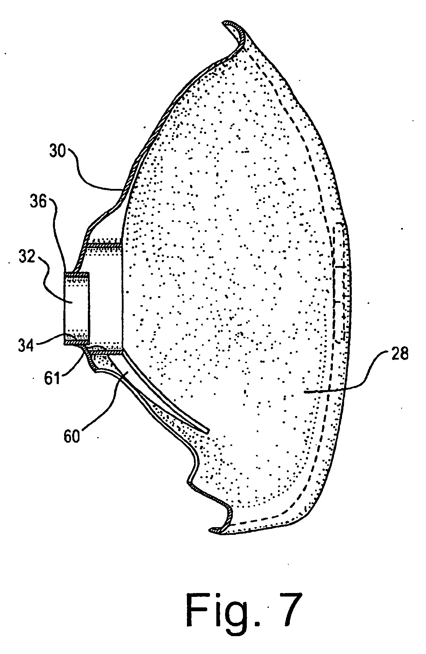

The nebulizer mask comprises a mask body, having a generally concave shape for fitting over the nose and mouth of a patient. The overall dimensions of the mask body will vary depending on its intended use, for example a mask intended for pediatric use will have reduced dimensions relative to an adult mask. The mask body (10) comprises a resilient flexible plastic such as PVC. Optionally, the mask body is transparent. When viewed from the front or rear, for example as seen in Figures 3 and 4, the mask has a shape and configuration for conforming closely to the patient's face, such that when worn by the patient the mask covers the nose and mouth orifices. The mask comprises upper (12) and lower (14) portions, with the upper portion being the portion of the mask body disposed above the central inlet (32) and the lower portion being disposed below this location. The upper and lower portions are defined herein solely for convenience of description; in practice, the mask body forms a continuous structure with no physical division between upper and lower portions. The lower portion (14) has in plan view, as seen in FIGURE 3, generally straight spaced apart parallel sides (16), rounded lower corners (18), and a substantially straight bottom margin (20). The upper portion of the mask is substantially triangular, having converged opposing sides (22) that meet at a rounded generally semi-circular apex (24). The mask has a soft pliable rim (26) for contacting the patient's face. The rim comprises a partially inverted portion of the mask body, forming a flange-like margin that provides a contact area, which forms an effective seal between the mask body and the patient's face. The mask body projects forwardly away from the patient's face, such that a concave interior space (28) is defined within the body to fit over the user's nose and mouth (see FIGURE 7). As will be discussed in more detail below, the mask body comprises a rounded wall that converges towards a generally central forwardly projecting region (30) of the mask where the inlet (32) enters the mask at a central opening (34).

The gas inlet tube (32) comprises a short tube that enters the mask body through the central opening (34). Preferably, the tube is cemented within the central opening (34) for a gas-tight non-slip seal. The inlet tube (32) projects forwardly from the mask body to form a flange (36). An elbow-shaped connector tube (38) connects to the flange (36) and projects outwardly therefrom. The connector tube (38) comprises an upper horizontal tube (40) disposed in a horizontal direction (when the mask is upright) which is co-axial with the inlet tube (32), for directing a flow of gas in a generally horizontal direction directly at the patient's nose and mouth region. The horizontal tube merges with a vertically oriented tube (42) which depends downwardly, for attachment to a nebulizer gas hose (100). The vertical tube (42) terminates at its lower end in a fitting or attachment means (43) for securely and quickly engaging the elbow to a hose or tube (100) leading from a nebulizer or atomizer. The fitting (43) is contemplated to comprise a conventional releasable attachment, but in practice it may comprise any convenient attachment, include any suitable permanent and releasable attachment.

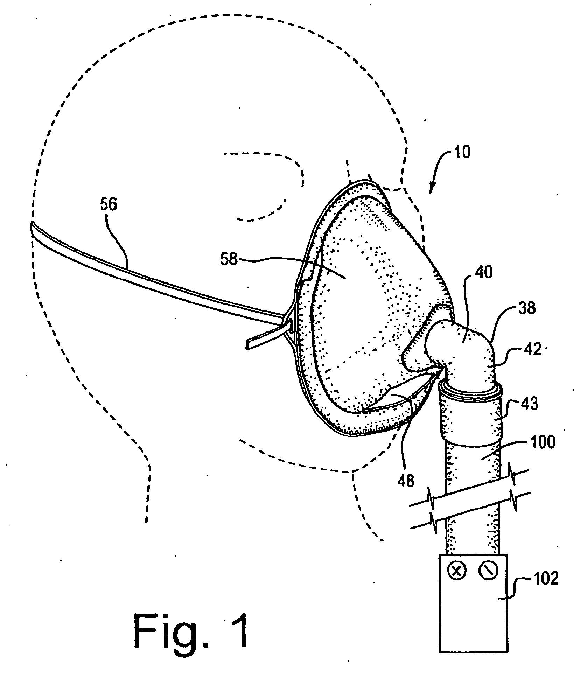

As seen in Figure 1, the gas hose (100) is operatively connected to a nebulizer (102), which is shown schematically and may constitute any suitable type of system for delivering a stream of drug-laden gas under pressure.

Returning to the mask body (10), the walls thereof are generally curved such that they converge at the inlet (32). By virtue of the curvature of the walls, all or a substantial portion of the walls curve inwardly towards the inlet (32), although a small portion of the wall adjacent to the rim (26) on either side of the mask body may flare outwardly to accommodate the wearer's cheek.

The rim has a configuration that permits it to conform to the majority of human faces. For this purpose, the rim angles slightly forwardly away form the patient's face at the upper end of the mask, where the rim is positioned over the bridge of the patient's nose, to accommodate the usually forwardly projecting nose bridge. In a like fashion, the lowermost edge of the rim may also slightly angle forwardly, where the mask rests on the upper chin of a patient, which also projects slightly forwardly.

A central lower opening (48) is provided within the lower portion (14) of the mask body, at a position directly below the central opening (34) when the mask is upright. In the embodiment described herein, the lower opening (48) is the sole opening within the mask body, apart from the rear face, which is fully open for sitting over the patient's face when the mask is in use. The lower opening (48) is thus made sufficiently large to permit an easy outflow of gases, both from the patient's own breath and the nebulized gas that is not inhaled by the patient. For example, the opening may have a width of about 3 cm and a height of about 2.5 cm, although these dimensions are presented only by way of an illustrative example, and other dimensions are contemplated and possible. It is contemplated that the mask may include additional embodiments.

As seen in FIGURES 3 and 7, an array of rearwardly projecting ribs (60) may be provided, radiating outwardly from the central region, in order to stiffen the mask body and assist in the fluid dynamic performance of the mask body. Preferably, three equally spaced ribs are provided. The ribs project rearwardly towards the user's face. The height of the ribs tapers outwardly, with their minimum height being at their end remote from the central region. The ribs may connect with a generally triangular wall (61) that surrounds the opening (34).

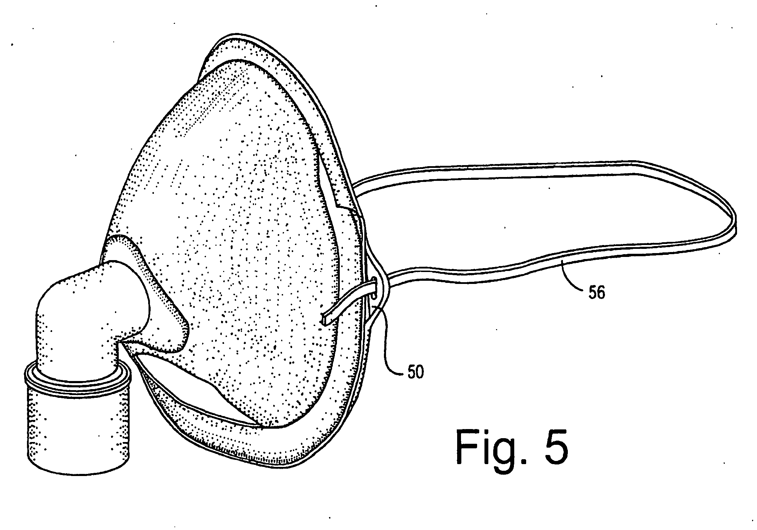

Opposing tabs (50) may be provided at or adjacent to the mask rim, in order to provide secure attachment points for a headgear (56) such as an elastic headband or other such securing means, for securing the mask to a patient's face as shown in Fig. 5.

The upper portion of the mask body, adjacent the curved upper end thereof, has an inward pinch (58) on either opposing side thereof (see FIGURE 1), which indents the mask body inwardly towards the wearer's face. This has the effect of shaping the mask body to generally conform to a human face and specifically the recessed facial area on either side of the upper nose region of the patient. This has the effect of maintaining a generally consistent spacing between the mask body and the patient's face, which is believed to improve the gas flow characteristics within the mask interior. This is intended to promote the gases to downwardly towards the lower opening and away from the upper rim, such that little or no gases leak around the mask rim and towards the patient's eyes or into the ambient air.

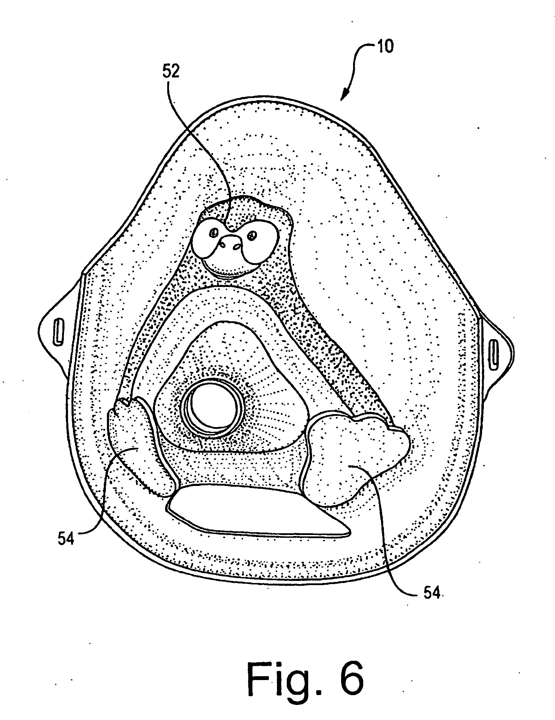

According to another aspect, as seen in FIGURE 6, the mask may be specifically adapted for pediatric use. For this purpose, the mask is provided in a reduced size suitably scaled for fitting over the face of an infant. The front of the mask is provided with a decorative element on the mask body, for example surrounding the central opening. By way of an illustrative example as shown in Figure 6, a cartoon of an animal such as a penguin may be provided, by molding animal-like features into the mask body, such as a face (52) and feet (54), together with coloring portions of the mask body to resemble a penguin or other animal, or a clown, or the like. This is intended to improve the acceptance of the mask for younger patients. It also serves the additional benefit of permitting medical staff to easily and quickly recognize a pediatric mask from one intended for adults and thereby avoid inadvertently applying an incorrectly shaped mask, which may have the unwanted effect of permitting excess gas leakage if the mask is too big.

Testing has been performed in order to compare the performance of an embodiment of the present mask against prior art designs.

While not wishing to be restricted to any particular theory of operation, it is believe that the present mask achieves a superior level of performance by the following means. Gases delivered through the nozzle are discharged in a generally horizontal direction (when the mask is upright) towards the patient's face in a direction that is perpendicular to the plane of the patient's face. That is, the gas thus delivered through the nozzle will tend to contact the patient's face at a perpendicular or somewhat oblique angle. Gas that is not inhaled by the patient will tend to rebound from the patient's face. The rebounded gas will either directly exit the lower opening, or contact the inside surface of the mask. Since all or substantially all of the mask wall is curved so as to taper towards a central point, tests have shown that gases within the mask that are imparted with an initial velocity from the central nozzle will tend to form one or more generally helical gas flows within the mask. The gas travelling in a helical path will tend to exert only minimal pressure at the rim of the mask, with the result that little or no gas is forced outwardly from the mask at the rim/face junction. Rather, all or substantially all of the gas will rapidly exit the mask at the lower opening, to be directed away from the mask in a substantially downward direction. This downwardly directed gas will tend to flow away from the patient's face, and particularly eyes. Further, since drug-laden gases tend to be heavier than air, the gases will tend to continue flowing downwardly, with little diffusion of the discharged gasses into the ambient air in the regions near the patient's face or at a breathing level of a caregiver within the room.

The following describes testing conducted on a representative sample of the mask, and comparing its performance to prior art commercial products. The tested example of the invention is referred to as the "OxyKid™" mask. The prior art mask used as a comparison in these examples is referred to as the KidsMed ™ mask.

### Example 1

The objective of this investigation was to compare the total dose, mass median aerodynamic diameter (MMAD), geometric standard deviation (GSD), respirable mass fraction (0.5 - 5 µm), respirable mass and treatment time of the OxyKid™ mask (an embodiment of the mask of the present invention) to the KidsMed™ Dragon Aerosol Mask (prior art mask) when operating under conditions of adult simulated breathing and while aerosolizing albuterol sulfate (2.5 mg/3 ml) with a Micromist™ nebulizer.

A simulated adult breathing pattern was created by alternately turning on an inhalation or exhalation valve and maintaining a constant flow of gas through each valve when open. A frequency generator was connected to a valve controller and the exhalation valve was connected to a compressor. The inhalation valve was connected to a vacuum source and a cascade impactor. The inhalation valve was adjusted to a flow rate of 28 liters per minute while connected to the cascade impactor and in the open condition. Similarly, the exhalation valve was adjusted to a flow rate of 18.7 liters per minute when in the open condition. The two valves were connected to a 22 mm wye connector. A 6 inch length of 22 mm corrugated tubing was placed through the back side of the mouth opening of an aerosol mask mannequin head. The mask was connected to the mannequin head and attached to a nebulizer. The frequency generator was set to an inspiratory time of 1.28 seconds and an exhalation time of 1.92 seconds as verified with an oscilloscope.

Each mask was tested with the same Micromist™ nebulizer while aerosolizing a standard dose of albuterol sulfate (2.5 mg/3 ml). Each mask was tested three times for a total of six tests. The nebulizer was connected to compressed air and operated at 6 liters per minute. Prior to starting the nebulizer, the valve controller was turned on and the inhalation/exhalation flow rates were verified. Sampling was performed with the cascade impactor during inhalation. Exhalation gases were forced out of the nebulizer as intended by the design of the nebulizer. The aerosol ambient scavenging system was positioned 1 to 2 inches away from the exhalation port of the nebulizer. Treatment time was measured and treatment is determined to have ceased when visual indication of aerosol production has ceased for a period of at least one second. After filling the nebulizer with 2 ml of medication, the initial weight of the nebulizer was measured. Upon completion of the simulated nebulizer treatment, the nebulizer and valve controller was turned off and the cascade impactor was disassembled. Specimen plates and the membrane filter for each stage of the cascade impactor were placed into different specimen containers. A calibrated pipette was used to place 10 ml of water into each specimen container. Concentration readings for each impactor stage were obtained using standard spectrophotometer techniques and the mass of drug deposited on each impactor stage was calculated. Upon completion of nebulization, a final weight of the nebulizer was obtained. The actual quantity of medication that remained in the nebulizer was calculated from the gravimetric change in the weight of the nebulizer, assuming normal nominal evaporation rates.

The cascade impactor data was entered into a spreadsheet and the accumulated mass percents were plotted on a log-log scale. Expulsion, MMAD, GSD, respirable mass fraction (0.5 to 5 µm) and respirable mass were determined using standard cascade impactor data analysis techniques. The data were tabulated and a statistical comparison was performed.

Table 1 shows the total dose delivered by the two different masks under conditions of simulated adult breathing and while aerosolizing 2.5 mg/3 ml of albuterol sulfate. The statistical analysis indicates that the difference in performance of the two masks was statistically significant.

**Table 1**

| | Aerosol Mask with Micromist™ | |
|---|---|---|
| | Nebulizer - Total Delivered Dose | |
| Nebulizer | OxyKid™ | KidsMed™ |
| Sample/ Statistics | Mask (µg) | Mask (µg) |
| 1 | 305 | 213 |
| 2 | 285 | 233 |
| 3 | 301 | 232 |
| Mean | 296.9 | 225.9 |
| Standard Deviation | 10.5 | 11.0 |

The results of the Student's T Test, two tailed comparison statistical analysis are shown below:

| | |
|---|---|
| Mean Difference | 71.1 |
| T Stat. | 8.10 |
| T (p = 0.05) | 2.78 |
| Diff. (Statistically Significant) | YES |

Table 2 shows the particle size delivered by the two masks under conditions of simulated adult breathing and while aerosolizing 2.5 mg/3 ml of albuterol sulfate. The statistical analysis indicates that the difference in performance of the two masks was not statistically significant.

**Table 2**

| | Aerosol Mask with Micromis™ | |
|---|---|---|
| | Nebulizer - Particle Size, MMAD (microns) | |
| Nebulizer Sample / Statistics | OxyKid™ | KidsMed™ |
| | Mask (microns) | Mask (microns) |
| 1 | 1.8 | 1.6 |
| 2 | 1.7 | 1.7 |
| 3 | 1.7 | 1.6 |
| Mean | 1.73 | 1.63 |
| Standard Deviation | 0.06 | 0.06 |

The results of the Student's T Test, two tailed comparison statistical analysis are shown below:

| | |
|---|---|
| Mean Difference | 0.10 |
| T Stat. | 2.12 |
| T (p = 0.05) | 2.78 |
| Diff. (Statistically Significant) | NO |

Table 3 shows the geometric standard deviation delivered by the two masks under conditions of simulated adult breathing and while aerosolizing 2.5 mg/3 ml of albuterol sulfate. The statistical analysis indicates that the difference in performance of the two masks was not statistically significant.

**Table 3**

| | Aerosol Mask with Micromist™ | |
|---|---|---|
| | Nebulizer - Geometric Standard Deviation (GSD) | |
| Nebulizer Sample / Statistics | OxyKid™ Mask | KidsMed™ Mask |
| 1 | 2.2 | 2.4 |
| 2 | 2.6 | 2.5 |
| 3 | 2.4 | 2.4 |
| Mean | 2.39 | 2.45 |
| Standard Deviation | 0.19 | 0.02 |

The results of the Student's T Test, two tailed comparison statistical analysis are shown below:

| | |
|---|---|
| Mean Difference | 0.06 |
| T Stat. | 0.51 |
| T (p = 0.05) | 2.78 |
| Diff. (Statistically Significant) | NO |

Table 4 shows the respirable fraction delivered by the two masks under conditions of simulated adult breathing and while aerosolizing 2.5 mg/3 ml of albuterol sulfate. The statistical analysis indicates that the difference in performance of the two masks was not statistically significant.

**Table 4**

| | Aerosol Mask with Micromist™ | |
|---|---|---|
| | Nebulizer - Respirable Fraction | |
| Nebulizer Sample / Statistics | OxyKid™ Mask (%) | KidsMed™ Mask (%) |
| 1 | 79 | 79 |
| 2 | 77 | 77 |
| 3 | 74 | 77 |
| Mean | 76.7 | 77.7 |
| Standard Deviation | 2.5 | 1.2 |

The results of the Student's T Test, two tailed comparison statistical analysis are shown below:

| | |
|---|---|
| Mean Difference | 1.0% |
| T Stat. | 0.63 |
| T (p = 0.05) | 2.78 |
| Diff. (Statistically Significant) | NO |

Table 5 shows the respirable dose delivered by the two masks under conditions of simulated adult breathing and while aerosolizing 2.5 mg/3 ml of albuterol sulfate. The statistical analysis indicates that the difference in performance of the two masks was statistically significant.

**Table 5**

| | Aerosol Mask with Micromist™ | |
|---|---|---|
| | Nebulizer - Respirable Dose (µg 0.5 - 5 microns) | |
| Nebulizer Sample / Statistics | OxyKid™ Mask (µg) | KidsMed™ Mask (µg) |
| 1 | 241 | 168 |
| 2 | 219 | 179 |
| 3 | 223 | 178 |
| Mean | 227.7 | 175.3 |
| Standard Deviation | 2.5 | 6.0 |

The results of the Student's T Test, two tailed comparison statistical analysis are shown below:

| | |
|---|---|
| Mean Difference | 52.3 |
| T Stat. | 7.06 |
| T (p = 0.05) | 2.78 |
| Diff. (Statistically Significant) | YES |

Table 6 shows the treatment time delivered by the two masks under conditions of simulated adult breathing and while aerosolizing 2.5 mg/3 ml of albuterol sulfate. The statistical analysis indicates that the difference in performance of the two masks was not statistically significant.

**Table 6**

| | Aerosol Mask with Micromist™ | |
|---|---|---|
| | Nebulizer - Treatment Time (minutes) | |
| Nebulizer Sample / Statistics | OxyKid™ Mask (minutes) | KidsMed™ Mask (minutes) |
| 1 | 11.8 | 11.5 |
| 2 | 11.5 | 10.3 |
| 3 | 11.5 | 10.8 |
| Mean | 11.58 | 10.83 |
| Standard Deviation | 0.14 | 0.63 |

The results of the Student's T Test, two tailed comparison statistical analysis are shown below:

| | |
|---|---|
| Mean Difference | 0.75 |
| T Stat. | 2.01 |
| T (p = 0.05) | 2.78 |
| Diff. (Statistically Significant) | NO |

All equipment met its specification before and after testing. There were no significant experimental variances. The measured medication delivered during simulated adult breathing was higher when using the OxyKid™ mask than the KidsMed™ mask. The statistical analysis shows that, at a confidence level of 95% it can be concluded that the OxyKid™ mask delivers more aerosolized drug than does the KidsMed™ mask.

It will be seen that the present invention has been described by way of preferred embodiments of various aspects of the invention. However, it will be understood that it is within the skill of those of ordinary skill in the art to modify or make workshop changes to the embodiments described herein, including substituting equivalent elements for those described herein. One may thus depart from the embodiments described in detail herein, while still remaining within the scope of the invention as defined in this patent specification as a whole, including the claims thereto.

## Claims

1. A mask for delivery of an aerosolized or nebulized drug in the form of a drug-laden gas delivered under pressure to a patient, said mask comprising a mask body (10) having a rim (26) for contacting a patient's face, said mask body (10) configured to substantially cover the nose and mouth of said patient when worn by a patient and to provide an interior space between said mask body (10) and said patient's face, said mask body (10) comprising a central inlet (32, 34) which is generally centered within the mask body (10) over the patient's nose for connection to a source of gas, an upper portion (12) of said mask body (10) above said inlet (32, 34), with said upper portion (12) of said mask body (10) being solid with no openings therein, and a lower portion (14) of said mask body (10) below said inlet (32, 34),
wherein said mask body (10) further comprises an opening (48) within said lower portion (14) of said mask body (10) configured to permit outflow of gases from said mask in a generally downward direction, wherein said opening (48) constitutes the sole ventilation opening within said mask, and
a fitting (36) projecting horizontally outward from said inlet (32, 34) for attachment to a gas conduit (100) for directing a generally horizontal flow of gas towards said patient's face, wherein gas is discharged from said inlet (32, 34), and
wherein said mask body (10) is curved between said rim (26) and said centrale inlet (32, 34) so as to taper towards the central inlet (32, 34),
**characterized in that**
said mask body (10) comprises an array of ribs (60) radiating outwardly from said inlet (32, 34) and projecting rearwardly from a rear surface of said mask body (10) into said interior space towards the face of said patient;
the sole opening (48) is located between first and second ribs (60) that project rearwardly from the rear surface of the lower portion (14) of the mask body (10), and
a third rib (60) projects rearwardly from the rear surface of the upper portion (12) of the mask, wherein portions of the mask body between the third rib (60) and the second rib (60) and between the third rib (60) and the first rib (60) are solid with no openings therein.

2. The mask of claims 1 wherein said rim (26) is contoured to form a seal between the mask body and the patient's face.

3. The mask of any of claims 1 to 2, wherein said mask body is indented (58) towards the patient's face on opposing sides of said mask body adjacent to an upper end thereof.

4. The mask of claim 1 wherein said opening (48) is substantially opposed to said patient's mouth.

5. The mask of claim 1 wherein the first through third ribs (60) are equally-spaced.

6. The mask according to claim 1, configured to fit a pediatric patient, said mask further including a decorative element (52, 54) molded therewithin.

7. The mask of claim 1 wherein said fitting (36) communicates with an elbow-shaped tube (38), one end of which connects with said fitting (36) and an opposed end of which depends downwardly for connection with a delivery tube (100) for delivery of a drug-laden gas.

8. The mask of any of claims 1 to 7, further comprising a generally triangular wall (61) surrounding said inlet (32, 34).

9. A system for delivery of an aerosolized or nebulized drug comprising a mask (10) In combination with a drug delivery system comprising a source of drug-laden pressurized gas (102) and a conduit (100) connecting said source to said mask, **characterized by** said mask comprising the mask as defined in any of claims 1 through 8.

## Patentansprüche

1. Maske zur Abgabe eines aerosolisierten oder zerstäubten Arzneimittels in Form eines arzneimittelhaltigen Gases, das unter Druck an einen Patienten abgegeben wird, wobei die Maske einen Maskenkörper (10) mit einem Rand (26) zum Kontakt mit dem Gesicht eines Patienten umfasst, wobei der Maskenkörper (10) dazu ausgestaltet ist, die Nase und den Mund des Patienten im Wesentlichen zu bedecken, wenn er von einem Patienten getragen wird, und einen Innenraum zwischen dem Maskenkörper (10) und dem Gesicht des Patienten vorzusehen, und wobei der Maskenkörper (10) einen mittigen Einlass (32, 34), der allgemein im Maskenkörper (10) über der Nase des Patienten zentriert zur Verbindung mit einer Gasquelle angeordnet ist, einen oberen Abschnitt (12) des Maskenkörpers (10) oberhalb des Einlasses (32, 34), wobei der obere Abschnitt (12) des Maskenkörpers (10) massiv ist und keine Öffnungen aufweist, und einen unteren Abschnitt (14) des Maskenkörpers (10) unterhalb des Einlasses (32, 34) umfasst,
- wobei der Maskenkörper (10) ferner eine Öffnung (48) im unteren Abschnitt (14) des Maskenkörpers (10) umfasst, die dazu ausgestaltet ist, ein Ausströmen von Gasen aus der Maske in einer allgemein abwärts verlaufenden Richtung zu ermöglichen, wobei die Öffnung (48) die einzige Ventilationsöffnung in der Maske darstellt, und
- ein Anschlussstück (36), das zur Befestigung einer Gasleitung (100) von dem Einlass (32, 34) horizontal nach außen hervorragt, um einen allgemein horizontalen Gasstrom zum Gesicht des Patienten zu lenken, wobei Gas aus dem Einlass (32, 34) ausströmt, und
- wobei der Maskenkörper (10) zwischen dem Rand (26) und dem mittigen Einlass (32, 34) gekrümmt ist, so dass er sich zum mittigen Einlass (32, 34) hin verjüngt, **dadurch gekennzeichnet, dass**
- der Maskenkörper (10) eine Anordnung von Rippen (60) umfasst, die vom Einlass (32, 34) strahlenförmig nach außen verlaufen und von einer Rückseite des Maskenkörpers (10) nach hinten in den Innenraum zum Gesicht des Patienten vorspringen,
- sich die einzige Öffnung (48) zwischen der ersten und der zweiten Rippe (60) befindet, die von der Rückseite des unteren Abschnitts (14) des Maskenkörpers (10) nach hinten vorspringen, und
- eine dritte Rippe (60) von der Rückseite des oberen Abschnitts (12) der Maske nach hinten vorspringt, wobei die Abschnitte des Maskenkörpers zwischen der dritten Rippe (60) und der zweiten Rippe (60) und zwischen der dritten Rippe (60) und der ersten Rippe (60) massiv sind und keine Öffnungen aufweisen.

2. Maske nach Anspruch 1,
bei der der Rand (26) profiliert ist, um zwischen dem Maskenkörper und dem Gesicht des Patienten eine Abdichtung zu bilden.

3. Maske nach Anspruch 1 oder 2,
bei der der Maskenkörper auf gegenüberliegenden Seiten des Maskenkörpers nahe einem oberen Ende desselben zum Gesicht des Patienten hin eingebuchtet (58) ist.

4. Maske nach Anspruch 1,
bei der die Öffnung (48) dem Mund des Patienten im Wesentlichen gegenüber liegt.

5. Maske nach Anspruch 1,
bei der die erste bis dritte Rippe (60) mit gleichem Abstand voneinander angeordnet sind.

6. Maske nach Anspruch 1,
die dafür ausgestaltet ist, einem pädiatrischen Patienten zu passen, wobei die Maske ferner ein darin eingeformtes dekoratives Element (52, 54) umfasst.

7. Maske nach Anspruch 1,
bei der das Anschlussstück (36) mit einer knieförmigen Röhre (38) in Verbindung steht, deren eines Ende mit dem Anschlussstück (36) verbunden ist und deren entgegengesetztes Ende zur Verbindung mit einer Abgaberöhre (100) zur Abgabe eines arzneimittelhaltigen Gases nach unten herabhängt.

8. Maske nach einem der Ansprüche 1 bis 7,
die ferner eine allgemein dreieckige Wand (61) umfasst, die den Einlass (32, 34) umgibt.

9. System zur Abgabe eines aerosolisierten oder zerstäubten Arzneimittels, das eine Maske (10) in Kombination mit einem Arzneimittelabgabesystem umfasst, welches eine Quelle eines arzneimittelhaltigen unter Druck stehenden Gases (102) und eine Leitung (100) aufweist, die die Quelle mit der Maske verbindet, **dadurch gekennzeichnet, dass** die Maske die in einem der Ansprüche 1 bis 8 definierte Maske umfasst.

## Revendications

1. Masque pour la délivrance d'un médicament aérosolisé ou nébulisé sous la forme d'un gaz chargé en médicament délivré sous pression à un patient, ledit masque comprenant un corps de masque (10) ayant un rebord (26) pour un contact avec le visage d'un patient, ledit corps de masque (10) configuré pour sensiblement recouvrir le nez et la bouche dudit patient lorsqu'il est porté par un patient et pour créer un espace intérieur entre ledit corps de masque (10) et le visage dudit patient, ledit corps de masque (10) comprenant une entrée centrale (32, 34) qui est de façon générale centrée dans le corps de masque (10) sur le nez du patient pour une connexion à une source de gaz, une partie supérieure (12) dudit corps de masque (10) au-dessus de ladite entrée (32, 34), avec ladite partie supérieure (12) dudit corps de masque (10) étant pleine sans ouverture dans celle-ci, et une partie inférieure (14) dudit corps de masque (10) en-dessous de ladite entrée (32, 34),
dans lequel ledit corps de masque (10) comprend en outre une ouverture (48) dans ladite partie inférieure (14) dudit corps de masque (10) configurée pour permettre un flux de sortie de gaz dudit masque dans un sens de façon générale vers le bas, dans lequel ladite ouverture (48) constitue l'unique ouverture de ventilation dans ledit masque, et
un raccord (36) se projetant horizontalement vers l'extérieur à partir de ladite entrée (32, 34) pour une fixation à une conduite de gaz (100) pour diriger un flux de façon générale horizontal de gaz vers le visage dudit patient, dans lequel le gaz est déchargé à partir de ladite entrée (32, 34), et
dans lequel ledit corps de masque (10) est courbe entre ledit rebord (26) et ladite entrée centrale (32, 34) de manière à se rétrécir vers l'entrée centrale (32, 34),
**caractérisé en ce que**
ledit corps de masque (10) comprend un ensemble de nervures (60) rayonnant vers l'extérieur à partir de ladite entrée (32, 34) et se projetant vers l'arrière à partir d'une surface arrière dudit corps de masque (10) jusque dans l'espace intérieur vers le visage dudit patient ;
l'ouverture unique (48) est située entre une première et une deuxième nervures (60) qui se projettent vers l'arrière à partir de la surface arrière de la partie inférieure (14) du corps de masque (10), et
une troisième nervure (60) se projette vers l'arrière à partir de la surface arrière de la partie supérieure (12) du masque, dans lequel des parties du corps de masque entre la troisième nervure (60) et la deuxième nervure (60) et entre la troisième nervure (60) et la première nervure (60) sont pleines sans ouverture dans celles-ci.

2. Masque selon la revendication 1, dans lequel ledit rebord (26) est conformé de façon à former une étanchéité entre le corps de masque et le visage du patient.

3. Masque selon l'une quelconque des revendications 1 à 2, dans lequel ledit corps de masque est gaufré (58) vers le visage du patient sur des côtés opposés dudit corps de masque adjacents à une extrémité supérieure de celui-ci.

4. Masque selon la revendication 1, dans lequel ladite ouverture (48) est sensiblement opposée à la bouche dudit patient.

5. Masque selon la revendication 1, dans lequel la première à la troisième nervures (60) sont également espacées.

6. Masque selon la revendication 1, configuré pour convenir à un patient pédiatrique, ledit masque incluant en outre un élément décoratif (52, 54) moulé dans celui-ci.

7. Masque selon la revendication 1, dans lequel ledit raccord (36) communique avec un tube en forme de coude (38), une extrémité duquel est connectée avec ledit raccord (36) et une extrémité opposée duquel est orientée vers le bas pour une connexion avec un tube de délivrance (100) pour délivrance d'un gaz chargé en médicament.

8. Masque selon l'une quelconque des revendications 1 à 7, comprenant en outre une paroi (61) de façon générale triangulaire entourant ladite entrée (32, 34).

9. Système de délivrance d'un médicament aérosolisé ou nébulisé comprenant un masque (10) en combinaison avec un système de délivrance de médicament comprenant une source de gaz sous pression chargé en médicament (102) et une conduite (100) connectant ladite source audit masque, **caractérisé par** ledit masque comprenant le masque selon l'une quelconque des revendications 1 à 8.
